Europäisches Patentamt

European Patent Office

Office européen des brevets

(19)

(11) Publication number: **0 322 151**

**A2**

(12) **EUROPEAN PATENT APPLICATION**

(21) Application number: 88311813.5

(22) Date of filing: 14.12.88

(51) Int. Cl.4: **C07C 49/813 , C07C 147/06 , C07C 147/10 , C08G 61/12 , C08G 67/00 , C08G 75/23**

(30) Priority: 23.12.87 GB 8730041
23.12.87 GB 8730042

(43) Date of publication of application:
28.06.89 Bulletin 89/26

(84) Designated Contracting States:
**DE FR GB IT NL**

(71) Applicant: **IMPERIAL CHEMICAL INDUSTRIES PLC**
**Imperial Chemical House Millbank**
**London SW1P 3JF(GB)**

(72) Inventor: **Colquhoun, Howard Matthew**
**8 Grove Park**
**Knutsford Cheshire WA6 8QA(GB)**
Inventor: **Lewis, David Frank**
**9 Blossom Heights Moss Farm**
**Hartford Northwich Cheshire CW8 4TF(GB)**

(74) Representative: Graham, John George et al
**Legal Dept. Patents PO Box 6 Bessemer Road**
**Welwyn Garden City Hertfordshire AL7 1HD(GB)**

(54) **Aromatic compounds.**

(57) Poly(thio)etherketones or- sulfones comprising repeating units (Ph-A-Ph₄-A-Ph). Starting products F-Ph-A-Ph₂-X and intermediates B-Ph-A-Ph₄-A-Ph-B. A is CO or SO₂, B is F or OH, X is CL or Br.

EP 0 322 151 A2

## Aromatic Compounds

This invention relates to aromatic compounds including aromatic ketones and sulphones and to processes for making them. The ketones and sulphones of the invention find particular, but not exclusive, use in preparation of aromatic polyketones and polysulphones. This invention further relates to such polyketones and polysulphones and to processes for their preparation and use.

In this specification, the term "Ph" is intended to refer to "1,4-phenylene".

According to a first aspect of the invention, there is provided an aromatic compound of formula I

FPhAPhPhX    I

wherein A is CO or SO$_2$ and is X is Cl or Br.

In a preferred process in accordance with a second aspect of the invention, preparation of the aromatic compound includes the step of reacting an acid of formula II

FPhAOH    II

or a Friedel-Crafts active derivative thereof with a biphenyl compound of formula III

HPhPhY    III

wherein Y is H, Cl or Br, in the presence of a Friedel-Crafts catalyst. Suitable Friedel-Crafts catalysts are ferric chloride, aluminium chloride, boron trifluoride, stannic chloride and antimony pentachloride but ferric chloride is preferred for sulphonylation and aluminium chloride for acylation. Preferably, a solvent is used in the acylation reaction, suitable solvents being 1,2-dichlorobenzene, 1,2,4-trichlorobenzene, 1,2-dichloroethane and nitrobenzene but 1,2-dichlorobenzene is preferred. If Y is H, the compound of formula I may be prepared in a subsequent step by halogenation, eg by electrophilic halogenation using a halogen in the presence of a Friedel-Crafts catalyst.

Alternative processes for preparation of the compound of formula I include the Friedel-Crafts reaction of the 4-nitro substituted acid of formula II or a Friedel Crafts active derivative thereof with the biphenyl compound III, halogenation if Y is H, followed by reduction of the nitro group to amino, diazotisation and fluorination.

According to a third aspect of the invention, there is provided an aromatic compound of formula IV

BPhAPh$_4$APhB    IV

wherein A is as hereinbefore defined and B is F or OH.

Preferably, in the compound of formula IV, each A is the same as the other. Also, preferably each B is the same as the other. Compounds of formula IV may be polycondensed to yield novel, useful polymers.

According to a fourth aspect of the invention, a process for the preparation of compounds of formula IV involves the step of coupling the compound of formula I and, when at least one B is to be OH, hydrolysing the product of the coupling reaction.

A preferred coupling reagent is ligand-stabilised, zero-valent nickel or other metal selected from the group: iron, cobalt, nickel. Triarylphosphine, preferably triphenyl phosphine, may be used as the ligand, the metal, which is provided as the halide or other salt, being reduced to the zero valent state by use of one or more electropositive metals such as magnesium, manganese or zinc. Coupling of the halogen compound with the zero-valent metal can be carried out catalytically in the presence of an excess of the electropositive metal. The reaction may be conveniently performed at temperatures within the range 10° to 100°C in a polar solvent such as a dialkylamide.

Alternatively, the coupling could be achieved via an Ullman reaction using finely - divided copper.

The product of the coupling rection may be hydrolysed to a bisphenol by refluxing the product in a solvent such as dimethyl sulphoxide and water in the presence of a strong base such as potassium hydroxide, followed by precipitation with acid and purification.

In an alternative process, the compound HPh$_4$H is reacted with an acid of formula II or a Friedel-Crafts active derivative thereof in the presence of a Friedel-Crafts catalyst. In yet another alternative process, the acid of formula HOAPh$_4$AOH or a Friedel-Crafts active derivative thereof is reacted with fluorobenzene in the presence of a Friedel-Crafts catalyst.

The catalyst may be any of those listed above, preferably ferric chloride or aluminium chloride.

According to a fifth aspect of the invention, there is provided an aromatic polymer comprising the unit of formula V

-PhAPh$_4$APh-    V

wherein A is as hereinbefore defined, linked to itself or to other units by ether and/or thioether linkages.

Such polymers may be prepared by polycondensing a compound of formula IV in the presence of a base. The polycondensation may be with a bisphenol, when each B is F, or a self-condensation when one B is F and the other B is OH, or a dihalo compound when each B is OH. The bisphenol may comprise a compound of formula IV when each B is OH. Thioether linkages can be obtained by substituting thiols or alkali sulphides or sulphoxides for some or all of the phenol and -group reactants.

Alternatively, the polymers may be prepared by polycondensing a compound of formula HOAPh₄AOH with a compound of formula HArOPhH. The polymerisation can be affected by dissolving the reactants, eg HOOCPh₄COOH and HPhOPhC(CF₃)PhOPhH, in HF/BF₃ or CF₃SO₃H; or by converting the acid to the acid chloride and using aluminium chloride as the catalyst.

Said other units may include groupings of formula Ar, wherein Ar is preferably selected from:-
1,4 -phenylene
1,3 -phenylene
4,4′ -biphenyl
3,3′ -biphenyl
2,2′ -biphenyl
4,4′ -benzophenone
4,4′ -diphenylsulphone
4,4′ -diphenylether
2,2′ -bis(4-phenylene)-propane
4,4′ -diphenyl-bis-(trifluormethyl)methane
2,7- naphthalene

Preferably, each A is the same as the other in the unit but may be the same or different from unit to unit.

In polymers according to the invention, preferably the proportion of aromatic rings in the polymer which is provided by the unit of formula V is at least 10%, more preferable at least 40% and, in particular, at least 60%.

In one preferred class of polymers according to the invention, A is CO. These polyetherketones may have the formula VI

BPhCOPh₄COPh[OArOPhCOPh₄COPh]$_n$B    VI

or may include transetherification products. Preferably, B is F.

Such polymers preferably have at least 60% of the aromatic rings in the polymer provided by the units of formula V and such polymers can have a Tg of at least 200°C and a Tg(°K)/Tm (°K) ratio which is at least 0.70. Also, such polymers can achieve substantially their highest attainable crystalline content when cooled from a molten state at a rate of at least 1°C/minute. Thus, these polymers exhibit surprising and advantageous properties in that they have a relatively high Tg but an unexpectedly low Tm (crystalline thermoplastics had been previously found to obey the rule that Tg (°K)/Tm (°K) is ½ to 2/3 (see Billmeyer, Textbook of Polymer Science, Wiley Interscience, 1962, 212)) and unusually high rates of crystallisation taking into account the comparative proximity of the Tg and Tm. Certain of the described polymers have been found to give essentially complete crystallisation on cooling at relatively high rates eg at least 1°C/minute and up to 20°C/min. Little further crystallisation has been found to occur on annealing or at lower rates of cooling. The polymer finds particular application in fabrication of articles for example composites, particularly carbon fibre composites, wherein rapid attainment of a high degree of crystallisation is advantageous. The low Tm facilitates fabrication of articles. The mechanical properties are retained up to relatively high temperatures, as indicated by the high Tg. Moreover the polymer exhibits a high degree of resistance to solvents and other hostile chemical environments on account of the high degree of crystallinity and a good resistance to burning and has a low rate of emission of toxic fumes and smoke on combustion.

According to a sixth aspect of the invention, a process for making a composite comprises the steps of:-
melting at least one such polymer of formula VI;
impregnating a fibrous material with the melted polymer;
cooling the composite at a rate greater than 1°C per minute to a temperature below the Tm of the polymer; and
allowing the composite to solidify to form a crystalline polymer matrix composite.

Preferred processes involve cooling at a rate greater than 4°C per minute.

The amount of fibrous material may be at least 20% by weight of short glass or carbon fibre; or 50 to 70%, preferably about 60% by volume of continuous glass or carbon fibre. A composite may be obtained by passing essentially continuous fibre, for example glass or carbon fibre, through molten polymer or a mixture containing it in a dissolved or finely dispersed state. The product obtained is a fibre coated with polymer and may be used alone, or together with other materials, for example a further quantity of the polymer, to form a shaped article. The production of compositions by this technique is described in more detail in EP-A 56703, 102158 and 102159.

Since the crystallinity of the polymer is developed to a maximum extent during cooling and/or any annealing, subsequent crystallisation, which may cause the article to suffer warping, cracking, other dimensional changes or other changes in physical properties, if rarely of ever encountered.

In another preferred class of polymers according to the invention, A is SO₂. These polyethersulphones may have the formula VII

BPhSO₂Ph₄SO₂Ph[OArOPhSO₂Ph₄SO₂Ph]$_n$B
VII

or may include transetherification products. Preferably, B is F.

Such polymers preferably have at least 60% of the aromatic rings in the polymer provided by units of formula V and such polymers can have a Tg of at least 240°C. Also, such polymers can be preferably crystalline or crystallisable. The term crystalline refers to a polymer which crystallises to a measurable extent on cooling from the melt. The term crystallisable refers to a polymer which crystallises to measurable extent on annealing above Tg but below Tm.

Thus. these polymers exhibit surprising and advantageous properties in that they are generally crystalline upon slow cooling from the melt or upon annealing and they exhibit very high glass transition temperatures, Tg.

The polymers exhibit good resistance to a wide range of solvents and other chemicals, retention of mechanical properties at high temperatures, good resistance to burning, emission of low proportions of toxic fumes and with low smoke on combustion.

However, as these polymers have a low rate of crystallisation, they may be quenched to give amorphous material processable at temperatures significantly below the melting point. The processed materials may then be annealed to give a crystalline polymer.

According to a seventh aspect of the present invention. a process for making an article comprises the steps of:-

melting at least one such polymer of formula VII;

forming the melted polymer into the configuration of said article;

quenching the article to a temperature below the melting point of the polymer;

allowing the quenched article to solidify to form an amorphous polymeric article;

heating the article to an annealing temperature between the Tg and crystalline melting point of the polymer; and

maintaining the article at the annealing temperature for a period sufficient to at least partially crystallise the polymer.

The crystallinity of the polymer is preferably developed to a maximum extent during annealing to minimise subsequent crystallisation which may cause the article to suffer warping, cracking, other dimensional changes or other changes in physical properties.

The invention also includes polymers having units in whcih A is CO and units in which A is $SO_2$ and or units in which, in the same unit, A is CO and $SO_2$, and transetherified polymers thereof.

Polymers in accordance with the invention, whetheer of formula VI or formula VII, can be melt processed into shaped articles, including films and insulating coatings on electrical conductors. They can be used in applications for which polyetherketones and or polyethersulphones have been proposed previously. for example for bearings or bearing liners or for other applications which require a combination of one or more of good electrical insulating properties, good resistance to a wide range of chemicals, retention of mechanical properties up to high temperature, good resistance to burning and the emission of low proportions of toxic fumes and with low smoke density on burning. Films whether undrawn, uniaxially-drawn or biaxially-drawn are especially useful when made of these polymers.

Whilst for many applications the polymers of the invention may be used with few if any additives, other than stabilisers, they may be used as polymer compositions containing one or more additives for example inorganic and organic fibrous fillers such as of glass. carbon or poly-paraphenylene terephthalamide: organic fillers such as polysulphones, polyketones, polyimides, polyester and polytetrafluorethylene at various levels of compatibility; and inorganic fillers such as graphite. boron nitride, mica, talc and vermiculite: nucleating agents; and stabilisers such as phosphates and combinations thereof.

Typically the total content of additives is 0.1 to 80%, preferably at most 70%, by weight of the total composition. The composition can contain for example 5 to 30% by weight of boron nitride: or at least 20% by weight of short glass or carbon fibre; or 50 to 70%, preferably about 60% by volume of continuous glass or carbon fibre; or a mixture of a fluorine-containing polymer, graphite and an organic or inorganic fibrous filler and the total proportion of these additives is preferably 20 to 50% by weight of the total composition.

The composition may be made by mixing the polymer with the additives for example by particle or melt blending. More specifically the polymeric material, in the form of dry powder or granules, can be mixed with the additives using a technique such as tumble blending or high speed mixing. The blend thus obtained may be extruded into a lace which is chopped to give granules. The granules can be subjected to a forming operation. for example injection moulding or extrusion, to give a shaped article.

Alternatively the composition may be film, foil powder or granules of the polymer with or without particulate additives, laminated with a fibrous filler in the form of mats or cloths.

The invention will now be described by reference to the following Examples.

Example 1 (Preparation of 4-bromo, 4'-(4-fluorobenzoyl)biphenyl)

A mixture of anhydrous $AlCl_3$ (21.8g). 4-bromobiphenyl (130 g), and 1,2-dichlorobenzene (600 cm$^3$) was stirred under nitrogen and 4-fluorobenzoyl chloride (88.4g) was added over 40 minutes, the reaction temperature being maintained at 34° C. The mixture was then heated to 65° and stirred for a further 5 hours, after which it was cooled to room temperature, poured into aqueous HCl (1 M, 2 1). A precipitate formed and was filtered off, washed with water and hexane, and dried to give a crude product (116.3 g). Recrystallisation from toluene/petroleum ether (bp 100-120° C) (1600 cm³, 1.3 v/v) followed by a second recrystallisation from acetone/2-butanone (3/1 v/v, 1770 cm³) yielded white crystals (50.3 g mp 183°) identified as [4-bromo-4'-(4"-fluorobenzoyl)-biphenyl] by $^{13}C$ nmr and mass spectrometry.

## Example 2 (Preparation of 4,4'-bis(4-fluorobenzoyl)-quaterphenyl)

A mixture of anhydrous $NiCl_2$ (2.6 g), triphenylphosphine (41.6 g), zinc dust (26 g) tetrabutylammonium iodide (28.4 g) and anhydrous dimethylacetamide (300 ml) was stirred at 60° under nitrogen for 45 minutes. To this was added, dropwise with stirring over 2 hours, a solution of FPhCOPhPhBr (37.5 g) in dimethylacetamide (360 ml). The resulting suspension was cooled to room temperature. The solid residue was washed with acetone until the washings were colourless, and then dried. This solid was extracted with aqueous nitric acid (1 1, 10% v/v) by stirring for 16 hours. The residue was filtered off, washed with water until it was acid-free, washed with acetone, and then dried. This material was recrystallised from 1,2,4-trichlorobenzene (3 litres) to give white crystals (mp 298°, 19.8 g) identified as [4,4'-bis(4"-fluorobenzoyl)quaterphenyl] by $^{13}C$ nmr and mass spectrometry.

## Example 3

A mixture of 4,4'-hexafluoroisopropylidene bisphenol ($HOPhC(CF_3)_2PhOH$) (1.784 g), FPhCOPh₄COPhF (3.068 g), anhydrous sodium carbonate (0.579 g) and diphenyl sulphone (30 g) was stirred under nitrogen and heated to 300° C over 1 hour. After stirring for a further 2½ hours at this temperature the mixture was cooled. The resulting solid was milled to a coarse powder (particle size 0.1-0.5 mm). Extraction with acetone (200 cm³), water (200 cm³) and again with acetone (200 cm³), all at reflux, afforded an off-white polymer (3.72 g) of inherent viscosity 0.78 ($CF_3SO_3H$ solution). The polymer was crystalline and unaffected by organic solvents. It was swollen by concentrated sulphuric acid but dissolved only by trifluoromethanesulphonic acid. Characterisation of the polymer by DSC showed a glass transition (onset) of 247° C and a sharp crystalline melting point at 364° C ($\Delta H$ fusion = 24 J/g) (Tg (°K)/Tm-(°K) = 0.82). On cooling the molten polymer, a crystallisation peak (Tc) was observed at 356°. The polymer has the structure:

FPhCOPh₄COPh[OPhC(CF₃)₂PhOPhCOPh₄COPh]-ₙF

The polymer was found to give essentially complete crystallisation on cooling at a rate of 20° C/minute. Little further crystallisation was found to occur on annealing or at lower rates of cooling.

## Example 4

The polymerisation described in Example 3 (in which a 5% molar excess of difluoride monomer over bis-phenol was used) was repeated using only a 2% molar excess (2.978 g) of the difluoride. The resulting polymer was of much greater molecular weight (IV 1.90 in $CF_3SO_3H$ solution) and characterisation by DSC showed a glass transition (onset) at 250° C. a sharp melting point at 360° C, and a crystallisation exotherm, on cooling, at 329° C (Tg-(°K)/Tm(°K) = 0.83).

## Example 5

A mixture of FPhCOPh₄COPhF (3.0 g, 5.449 mol), 4,4'-dihydroxy diphenylsulphone (1.328 g, 5.306 mol), anhydrous sodium carbonate (0.596 g, 5.623 mol) and diphenyl sulphone (30 g) was stirred under nitrogen and heated to 320° C over 1 hour. After an hour at this temperature, the temperature was raised to 340° C and stirred for a further 2 hours. Cooling and workup as in Example 3 gave 3.01 g of a polymer which was soluble in 98% sulphuric acid (IV = 0.38), and which, when characterised by DSC, showed a glass transition (onset) at 224° C, a melting endotherm at 411° C ($H_{fusion}$ = 38 J/g), and a crystallisation exotherm on cooling at 375° C (Tg(°K)/Tm(°K) = 0.73). The polymer has the structure FPhCOPh₄COPh-[OPhSO₂PhOPhCOPh₄COPh]ₙF

## Example 6

The polymerisation described in Example 3 was repeated except that 10 mole % of the FPhCOPh₄COPhF was replaced by ClPhSO₂Ph₂SO₂PhCl, and the ratio of bis-phenol to total dihalides was adjusted to give a 3% molar excess of dihalide over bis-phenol. The copolymer so obtained was of IV 1.11 ($CF_3SO_3H$ solution) and showed a glass transition (onset) at 255° C, a melting point of 327° C, and a crystallisation exotherm on cooling of 307° C (Tg(°K)/Tm(°K) = 0.88).

The polymers of Examples 4, 5 and 6 exhibited crystallinity characteristics similar to the polymer of Example 3.

Example 7

The polymerisation described in Example 4 was repeated except that 25 mole % of the FPhCOPh₄COPhF was replaced by ClPhSO₂Ph₂SO₂PhCl. The resulting copolymer was of IV 0.93 (CF₃SO₃H solution), and showed, by DSC, a glass transition (onset) at 227°C. No melting or crystallisation peaks were observed.

Example 8

The polymerisation described in Example 4 was repeated except that 25 mole % of the FPhCOPh₄COPhF was replaced by FPhSO₂PhF. The resulting copolymer was of IV 2.94 (CF₃SO₃H solution), and showed, by DSC a glass transition (onset) at 227°C and a broad, weak, melting endotherm at 270 -290°C (taking Tm = 280°C, Tg-(°K)/Tm(°K) = 0.90). No crystallistion exotherm was observed on cooling.

Example 9 (Preparation of 4-bromo-4′-(4-fluorobenzenesulphonylbiphenyl)

A mixture of 4-bromobiphenyl (202.6g), 4-fluorobenzenesulphonyl chloride (169.1g) and ferric chloride (1.0g, anhydrous) was heated with stirring under nitrogen to 85°C. After 15 mins the temperature was raised to 135°C for 1hr and then further raised to 190°C for 45 minutes. The reaction mixture was cooled and treated with toluene (500 cm³) and 2-ethoxyethanol (50 cm³). The resultant suspension was stirred under reflux for 2 hr. After cooling, the solid product was filtered, washed with toluene (200 cm³) and hexane (200 cm³) and dried under vacuum to give a white powder (275.5g). This was recrystallised from toluene to give white crystals of 4-bromo-4′-(4-fluorobenzenesulphonyl)-biphenyl (241.5g, mp 201°C) which was characterised by ¹³C nmr and mass spectrometry.

Example 10 (Preparation of FPhSO₂Ph₄SO₂PhF)

A mixture of nickel chloride (3.2g, anydrous), triphenylphosphine (51.94), zinc dust (32.5g), tetrabutylammonium iodide (35.5g) and dimethylacetamide (375 cm³) was stirred under nitrogen at 60°C for 1 hour. A solution of 4-bromo-4′-(4-fluorobenzenesulphonyl)biphenyl (55g) in dimethylacetamide (450 cm³) was added dropwise with stirring over 1 hr. The mixture was stirred at 60°C for 1hr, cooled to room temperature, filtered and the solid residue was washed with acetone (21) and dried. The solid was then stirred in dilute nitric acid (10% v v, 21, 16 hr), filtered, washed with water until acid free and dried to give a crude product (33.3g) which was crystallised from a mixture of 1,2,4-trichlorobenzene (6.21) and acetylacetone (20 cm³). The white crystalline product was filtered, washed with acetone and dried under vacuum. The product (31.2g, mp 421°C) was identified as

FPhSO₂Ph₄SO₂PhF

by ¹³C nmr and mass spectrometry.

Example 12

The monomer from the preceding experiment (3.436g), 4,4-dihydroxybenzophenone (1.137g), sodium carbonate (0.596g) and diphenylsulphone (30g) were stirred at 300°C under nitrogen for 2 hours, then stirred for a further 1 hour at 320°C. The mixture was cooled to room temperature, milled to a coarse powder and extracted successively under reflux with 200 cm³ portions of acetone, water and acetone. The resultant polymer (3.26g) was of inherent viscosity (in H₂SO₄) of 0.74 and was characterised by DSC as having Tg of 299°C and a crystalline melting point, Tm, of 373°C. Repeated DSC showed that the polymer had not crystallised on cooling but showed only a Tg at 277°C. Annealing at 340°C for 16 hr resulted in the polymer recovering ca 50% of the original crystallinity, raising the Tg to 287°C with the Tm at 372°C. The polymer was moulded at 400°C to give a tough, translucent film.

The polymerisation was repeated using alternative bisphenols with results set out in Table 1.

Example 11 (Preparation of HOPhSO₂Ph₄SO₂PhOH by hydrolysis of FPhSO₂Ph₄SO₂PhF)

A solution of FPhSO₂Ph₄SO₂PhF (20 g) and KOH (11.9 g) in a mixture of dimethyl sulphoxide (85 cm³) and water (21 cm³) was stirred at reflux (138°C) for 8½ hours under nitrogen. The cloudy solution was filtered through filter-aid to give a clear yellow filtrate which was diluted with water until the first trace of a permanent precipitate appeared. Acidification with 20 cm³ of concentrated hydrochloric acid gave a white precipitate which was filtered off, washed with hot water, and dried under vacuum to give 18.9 g of crude product. Recrystallisation from dimethylacetamide gave 14.8 g of white crystals, mp 396°C, identified as by mass spectrometry and $^{13}$C nmr as $HOPhSO_2Ph_4SO_2PhOH$.

The determination of Tg in the aforegoing Examples is by differential scanning calorimetry (DSC) carried out by examining a 10 mg sample of the polymer in a Mettler DSC 20 instrument, using a heating rate of 20°C/min under nitrogen. The onset of the Tg transition may be determined from the resultant curve. This is measured as the intersection of lines drawn along the pre-transition baseline and a line drawn along the greatest slope obtained during the transition.

## TABLE 1

Polysulphones prepared in accordance with Example 12 from 4,4'-bis(4-fluorobenzenesulphonyl)quaterphenyl

| Bis-phenol | IV | Tg (amorphous)° | Tg(amended)° annealed | Tm° (annealed) | ΔH fusion (J/g) (annealed) |
|---|---|---|---|---|---|
| HOPhCOPhOH (Example 12) | 0.74 | 277 | 287 | 371 | 12.7 |
| HOPhC(CF$_3$)$_2$PhOH | (insol in all solvents at room temp) | 275 | 285 | 351 | 14 |
| HOPhOPhOH | 0.54 | 259 | 265 | 364 | 25.7 |
| HO⬡⬡OH | 0.72 | 286 | – | – | – |
| HOPhOH | insol | – | – | 450 | 64.0 |
| HOPh$_2$OH | insol | – | – | 467 | 86.4 |
| HOPhCOPh$_2$COPhOH | 1.11 | 279 | 279 | – | – |
| HOPhCOPhOH : HOPh$_2$OH (1:1) | 0.62 | 284 | 297 | 418 | 27.4 |
| HOPhCOPhOH : HOPh$_2$OH (1:1) | 0.81 | 289 | 296 | 414 | 15.7 |

EP 0 322 151 A2

TABLE 1(continued)

| Bis-phenol | IV | Tg (amorphous)° (°C) | Tg(annealed) (°C) | Tm (annealed) (°C) | $\Delta H$ fusion (J/g) (annealed) |
|---|---|---|---|---|---|
| $HOPhSO_2PhOH$ | 0.52 | 294 | 294 | – | – |
| (HO—$\bigcirc$—$\bigcirc$—OH structure) | 0.57 | 256 | 256 | 375 | 20 |
| $HOPhSO_2PhOH$ + $HOPhPhOH$ (15:35) | 0.74 | 298 | 308 | – | – |
| $HOPhSO_2Ph_4SO_2PhOH$ | 0.51 | – | – | 521 | 39.9 |
| $HOPhSO_2Ph_4SO_2PhOH$ + $HOPhSO_2PhOH$ (1:1) | 0.96 | 317 | 318 | – | – |

## Claims

1. An aromatic compound of formula I

FPhAPhPhX    I

where Ph is 1,4-phenylene, A is CO or SO$_2$ and X is Cl or Br.

2. A process of making an aromatic compound of formula I

FPhAPhPhX    I

wherein Ph is 1,4-phenylene, A is CO or SO$_2$ and X is Cl or Br, comprising reacting an acid of formula II

FPhAOH    II

or a Friedel-Crafts active derivative thereof with a biphenyl compound of formula III

HPhPhY    III

wherein Y is H, Cl or Br, in the presence of a Friedel-Crafts catalyst.

3. A process of making an aromatic compound of formula I

FPhAPhPhX    I

wherein Ph is 1,4-phenylene, A is CO or SO$_2$ and X is Cl or Br, comprising reacting an acid of formula IIA

O$_2$NPhAOH    IIA

or a Friedel-Crafts active derivative thereof with a biphenyl compound of formula III

HPhPhY    III

wherein Y is H, Cl or Br, in the presence of a Friedel-Crafts catalyst, reducing the nitro group and diazotising and fluorinating the resultant amine group.

4. A process according to claim 2 or claim 3, which, when Y is H, comprises halogenating the product of the Friedel-Crafts reaction.

5. An aromatic compound of formula IV

BPhAPh$_4$APhB    IV wherein Ph is 1,4-phenylene, A is CO or SO$_2$ and B is F or OH.

6. A process for making a compound of formula IV

BPhAPh$_4$APhB    IV

where Ph is 1,4-phenylene, A is CO or SO$_2$ and B is F or OH, comprising coupling a compound of formula I

FPhAPhPhX    I,

where X is Cl or Br, and, when at least one B is to be OH, hydrolysing the product of the coupling reaction.

7. A process for making a compound of formula IV

BPhAPh$_4$APhB    IV

wherein Ph is 1,4-phenylene, A is CO or SO$_2$ and B is F or OH, comprising reacting the compound HPh$_4$H with an acid of formula II

FPhAOH    II

or a Friedel-Crafts active derivative thereof in the presence of a Friedel-Crafts catalyst and, when at least one B is to be OH, hydrolysing the product of the Friedel-Crafts reaction.

8. An aromatic polymer comprising a unit of formula V

-PhAPh$_4$APh-    V

wherein Ph is 1,4-phenylene and A is CO or SO$_2$, linked to itself or to other units by ether and/or thioether linkages.

9. An aromatic polymer according to claim 8, wherein A is CO, which has a Tg of at least 200° C and a Tg (°K)/ Tm (°K) ratio which is at least 0.70 and which achieves substantially its highest attainable crystalline content when cooled from a molten state at a rate of at least 1° C/minute.

10. An aromatic polymer according to claim 8, wherein A is SO$_2$, which is crystalline or crystallisable and which has a Tg of at least 240° C.

11. In aromatic compounds of formula IV as defined in claims 5 to 7, each A is the same as the other and, in aromatic polymers as defined in claim 8, each A is the same as the other in the unit but may be the same or different from unit to unit.

12. A process for making a com o e comprising the steps of:

melting a polymer according to claim 8, wherein A is CO;

impregnating a fibrous material with the melted polymer;

cooling the composite at a rate of greater than 1° C/min to a temperature below the Tm of the polymer; and

allowing the composite to solidify to form a crystalline polymer matrix composite.

13. A process of making an article comprising the steps of:

melting a polymer according to claim 8, wherein A is SO$_2$;

forming the melted article into the configuration of said article;

quenching the article to a temperature below the melting point of the polymer;

allowing the quenched article to solidify to form an amorphous polymeric article;

heating the article to an annealing temperature between the Tg and the crystalline melting point of the polymer; and

maintaining the article at the annealing temperature for a period sufficient to at least partially crystallise the polymer.